# EUROPEAN PATENT SPECIFICATION

(11) **EP 2 151 205 B1**
(45) Date of publication and mention of the grant of the patent: **18.01.2012**
(21) Application number: 09251935.4
(22) Date of filing: 04.08.2009
(51) Int. Cl.: A61B 17/30

(54) **Forceps**
Pinzette
Pincette

(30) Priority: 05.08.2008 GB 0814339
(43) Date of publication of application: 10.02.2010
(73) Proprietor: European First Aid Limited, Oxhey, Watford Hertfordshire WD19 4QQ (GB)
(72) Inventor: Poole, Terence James, Oxhey Watford Hertfordshire WD19 4QQ (GB)
(74) Representative: Mabey, Katherine Frances

(56) References cited:
- WO-A-95/07662
- WO-A-99/24091
- WO-A-2005/048854
- US-A- 4 452 106
- US-A- 5 156 431
- US-A- 5 472 450
- US-A1- 2008 249 547

## Description

The present invention relates to forceps, and more particularly forceps for picking up sharps e.g. needles and/or syringes. The invention related, to disposable plastic forceps i.e. forceps which intended to be used on a single occasion and disposed of, rather than being sterilised or cleaned for reuse.

There is an increasing a need to be able to safely handle sharps, i.e. needles and syringes in a range of contexts. Many sectors have a need to be able to safely handle sharps, including the police, emergency services, leisure industry, schools, colleges and shops. The sharps may be collected for disposal or to be retained for subsequent analysis or evidence purposes. For example, sharps may be collected from an area as part of a clean-up operation, e.g. after being discarded by drug users, or in any context where the discarded sharps may represent a high risk to other people. If sharps are found in a public area, such as a shop, school, or on a form of public transport, it is important to be able to retrieve the sharp without delay for disposal purposes, or in some cases, in order to be able to send the sharp for analysis to try to determine its origin, and the level of risk it may have presented. It is important that the operator should be able to handle the sharps in an efficient manner, which minimizes any risk to that person, and in manner which complies with the ever increasing body of health and safety legislation in this area. It will be appreciated that in these contexts, the sharps e.g. needles and syringes will generally need to be retrieved from a surface, e.g. the ground etc, which presents greater difficulties in terms of retrieval using conventional forceps.

While forceps have been previously been proposed for handling syringes, the Applicant has found that often it is desirable to be able to pick up needles which are unattached to a syringe. This may be necessary if a needle becomes separated from a syringe, or in order to be able to isolate a needle from a syringe for evidence or analysis purposes. This is often the case at locations where drug users have been active. However, conventional forceps which are designed for handling syringes are unsuitable for picking up needles. US 5, 156, 431 discloses forceps according to the preamble of claim 1. The present invention seeks to overcome these and other problems of conventional forceps.

In accordance with the present invention forceps as defined in claim 1 are provided ;

In accordance with the invention, the forceps therefore include a gripping area at the distal end of at least one of the arms of the pair of arms of the forceps, which gripping area has a first gripping profile to allow secure gripping of a syringe barrel, and a second gripping profile enabling secure gripping of a needle when the arms are manipulated together in use. It will be appreciated that the gripping area cooperates with an opposed gripping area at the distal end of the other arm of the forceps in use to grip objects when the arms are manipulated towards one another. The forceps are therefore configured to allow both needles and syringes to be picked up from a surface. The opposed gripping areas are preferably of the same construction i.e. both comprising the first and second gripping profiles.

In this manner, the forceps may be used to retrieve either a syringe, or, alternatively, a needle i.e. a single needle. As the gripping profiles are adapted to snugly fit respectively around the body/outer housing of a syringe, or the outer surface of a needle, both needles and syringes may be picked up safely and efficiently as required. This overcomes problems associated with conventional forceps configured only for picking up syringes. Any attempt to pick up needles from a surface using forceps intended for picking up much larger diameter syringes is a risky and inefficient operation, increasing the possibility of the user's hand inadvertently contacting the needle as they attempt to scoop up the needle, and the possibility of the needle being dropped again, with the risk that it may contact a part of the user's body, or another person. Any attempt to pick up a needle from a surface using unsuitable forceps may also result in excessive handling of the needle, e.g. when pushing it along a surface to try to get a grip on it, and/or if the needle is dropped and needs to be repeatedly retrieved. Such handling is undesirable as it may contaminate the needle, and destroy vital evidence if the needle is being collected for analysis or evidence purposes e.g. by the police.

The second gripping profile is located between the first gripping profile and the free distal end of the arm. By locating the second profile for gripping needles closer to the free end of the arm, interference by the first profile for gripping syringes with operation of the forceps to pick up needles may be avoided, especially when retrieving needles which are lying on a surface. In particular, this may avoid having to manipulate the forceps under a needle in a scooping motion to engage the needle with the second gripping profile when retrieving a needle from a surface. Such an action is undesirable, as discussed above, as it may increase the risk to the operator, and also may increase the risk of contaminating the needle. Preferably the first gripping profile is located adjacent the second gripping profile. This may minimize interference of the second gripping profile with operation of the first gripping profile to pick up syringes, especially in particularly preferred embodiments when the second gripping profile is located at or adjacent the free end of the arm.

In particularly preferred embodiments the first gripping profile is located directly inboard of the second gripping profile.

The centre of the first gripping profile may be located a distance of no more than 15 mm from the distal end of the arm. In embodiments the centre of the first gripping profile is located a distance of at least 2 mm from the end of the arm. In preferred embodiments the centre of the first gripping profile is located a distance of from 2.4 mm to 12.8 mm from the distal end of the arm, and preferably in the range of from 6 mm to 10 mm, most preferably from 7 mm to 9 mm. In some exemplary embodiments the centre of the first gripping profile is located a distance of 8.5 mm from the distal end of the arm. In embodiments the first gripping profile is preferably symmetrical i.e. about a line extending perpendicular to the axis of the arm.

The second gripping profile for gripping needles should be located appropriately to enable needles to be securely picked up in the second gripping profile in use. In preferred embodiments the second gripping profile is located at or adjacent the distal end of the arm. In particularly preferred embodiments the second gripping profile is located at the distal tip of the arm. This has been found to facilitate picking up of needles, particularly when lying on a surface, allowing the needle to be retrieved with a simple up and down type action, and without needing to resort to a scooping type action. When the second gripping profile is located adjacent or at the distal end of the arm the end of the arm may still slide under a needle to allow the needle to enter the second gripping profile without difficulty, and without interference from the first gripping profile. The distal end of the second gripping profile may be located in the range of less than 1 mm, less than 0.5 mm or less than 0.05 mm from the distal end of the arm.

The first gripping profile may be of any configuration which enables it to snugly fit around the outer surface of the barrel of a syringe, to enable gripping of the barrel when the gripping areas of the arms are brought together. The first gripping profile should therefore be configured to snugly fit around the outer surface e.g. circumference, of objects having dimensions typical of a syringe barrel. Syringes typically have an external diameter in the range of from 5 mm to 20 mm in the barrel region, although in some case, for example veterinary syringes, the barrel may have an external diameter in the range of up to 30 mm. Preferably the first gripping profile is therefore adapted to snugly fit around the outer surface of objects e.g. syringe barrels having an (external) width or diameter of at least 5 mm, preferably no greater than 30 mm, and more preferably no greater than 20 mm. In embodiments the first gripping profile is adapted to snugly fit around the outer surface of objects having a width or diameter most preferably in the range of from 5 mm to 30 mm, or from 5 mm to 20 mm.

The first gripping profile extends around at least a part of the outer surface e.g. circumference/perimeter of the syringe barrel in use to grip the syringe. The first gripping profile may be configured to extend around any suitable portion of the outer surface of a syringe body or barrel for this purpose, and the suitable extent will depend upon various factors, such as whether a cooperating matching gripping profile is provided on the facing gripping area of the other arm, the level of resilience in the material of the arm etc. The first gripping profile is preferably configured to extend around at least 25 % of the circumference of a syringe barrel having a diameter of 25 mm, more preferably at least 30%, and preferably at least 40% thereof. In embodiments the first gripping profile is adapted to extend around up to 50% of the circumference/perimeter of the outer surface of a syringe barrel having a diameter of 25 mm. In preferred embodiments the gripping area of the opposed arm has a matching first gripping profile which extends around the same proportion of the circumference of the syringe barrel. In this way the opposed first gripping profiles may together extend around at least 50%, more preferably at least 60% and most preferably at least 80% of the circumference of a syringe barrel having a diameter of 25 mm in use.

It will be appreciated that the first gripping profile is intended to grip a syringe such that the syringe extends transversely i.e. perpendicular to the longitudinal axis of the forceps, such that it will be generally horizontal when the forceps are generally vertical. Thus, the first gripping profile should extend in the longitudinal direction of the forceps/arm to engage around the outer surface of the syringe. The first gripping profile may extend a distance of at least 3 mm, preferably at least 5 mm, and preferably at least 10 mm along the length of the arm. The first gripping profile may extend a distance of no greater than 25 mm, or 20 mm or 16 mm along the length of the arm. In embodiments the first gripping profile extends a distance in the range of from 3 mm to 20 mm, or more preferably from 10 mm to 16 mm along the length of the arm. In exemplary embodiments the first gripping profile may extend a distance of around 13 mm along the length of the arm. These ranges are similar to the typical diameter of a syringe barrel, and therefore permit the gripping profile to snugly engage around a syringe barrel in use. The distance that the first gripping profile extends along the length of the arm may be referred to as the "width" of the first gripping profile. These values should be measured at the mouth of the profile i.e. at its front when the profile defines a depth.

Preferably the first gripping profile has a depth. This enables it to fit around the syringe barrel. Preferably the depth is in the range of at least 0.5 mm, and preferably at least 2 mm. Preferably the depth is no more than 5 mm, and preferably no more than 3 mm. Preferably the depth is in the range of from 0.5 mm to 5 mm, and preferably in the range of from 2 mm to 3 mm, and in exemplary embodiments is about 2.5 mm. In embodiments in which the depth of the first gripping profile varies i.e. if it is arcuate, the maximum depth should lie in the above ranges. This may be the depth at the centre of the first gripping profile. These ranges have been found to provide a gripping profile which cooperates well with the typical curvature/dimensions of syringe barrels.

The first gripping profile may be of any suitable configuration. For example elliptical. These are the shapes defined by the syringe engaging surface in vertical cross section i.e. along a line parallel to a longitudinal axis of the arm. If the first gripping profile is adapted to cooperate with a syringe which is not cylindrical, then the references above to "circumference" of the syringe may be understood to refer to its perimeter. However, most syringe barrels are generally cylindrical, and the first gripping profile is therefore preferably configured to fit around a cylindrical body. In any case the first gripping profile is arcuate. The surface is convexly curved to co-operate with the exterior surface of the syringe. In these embodiments the first gripping profile may define an arc. The arc preferably defines a central angle in the range of from 35 degrees to 60 degrees, and preferably of around 50 degrees. The arc may define an arc length of at least 3 mm, preferably at least 5 mm, and preferably at least 10 mm. The arc may define an arc length of no greater than 25 mm, or 20 mm or 16 mm. In embodiments the arc defines a length in the range of from 3 mm to 25 mm, from 5 mm to 20 mm, from 10 mm to 16 mm, and in embodiments of around 13 mm.

Preferably the first gripping profile defines a channel for receiving the syringe barrel. The channel extends across the gripping area. The channel may be said to have a width extending in the longitudinal direction of the arm. The channel width corresponds to the distance the profile extends along the longitudinal direction of the arm. The channel is arcuate, as discussed above. In these preferred embodiments, the channel is arc shaped, and may be semi-circular. Whatever the shape of the channel, in these preferred embodiments the channel preferably defines a width in the ranges discussed above for the distance the first gripping profile extends along the length of the arm. Preferably the channel has a width of at least 3 mm, at least 5 mm or at least 10 mm. Preferably the channel has a width of less than 25 mm, less than 20 mm, or less than 16 mm. The channel may have a width in the range of from 3 mm to 20 mm, preferably from 10 mm to 16 mm, and in embodiments, around 13 mm. The width is the distance the channel extends in the longitudinal direction of the arm measured at its mouth. The channel preferably has a depth of at least 0.5 mm, and preferably at least 2 mm. Preferably the channel has a depth of no more than 5 mm, or 3 mm. Preferably the channel has a depth in the range of from 0.5 mm to 5 mm, and preferably in the range of from 2 mm to 3 mm, and in embodiments may be about 2.5 mm.

The first gripping profile may define a substantially smooth surface for gripping the syringe barrel or a roughened surface. Preferably, the first gripping profile comprises a substantially smooth surface for gripping the syringe barrel. The gripping surface is that surface intended to contact the syringe barrel in use, i.e. that surface which forms the gripping profile and may have dimensions in the ranges given above.

It will be appreciated that the first gripping profile may define dimensions in any combination of the above ranges.

The second gripping profile may be of any configuration which enables it to snugly fit around the outer surface of a needle to enable gripping of the needle when the gripping areas of the arms are brought together. The second gripping profile should therefore be configured to fit around the outer surface e.g. circumference, of objects having dimensions typical of a needle. Typically needles may have an external diameter in the range of from 0.1 mm and 1.5 mm. Preferably the second gripping profile is therefore configured to snugly fit around the outer surface of an object having a diameter of no greater than 1.5 mm. Preferably the second gripping profile is configured to fit around the outer surface of an object having a diameter of at least 0.1 mm. Preferably the second gripping profile is configured to fit around the outer surface of an object having a diameter in the range of from 0.1 mm to 1.5 mm.

The second gripping profile may be configured to extend around any suitable portion of the diameter of a needle for this purpose, and the suitable extent will depend upon various factors, such as whether a cooperating matching gripping profile is provided on the facing gripping area of the other arm, the level of resilience in the material of the arm etc. Preferably the second gripping profile therefore has a maximum width or diameter corresponding to the typical diameter of a needle. Preferably the second gripping profile has a maximum width or diameter of no greater than 2 mm, and preferably no greater than 1.5 mm. Preferably the second gripping profile has a maximum width or diameter of at least 0.1 mm. Preferably the second gripping profile has a maximum width or diameter of in the range of from 0.1 mm to 2 mm, or from 0. 1 mm to 1.5 mm. These ranges are chosen to allow the profile to snugly fit around needles of the typical diameters described above. The width or diameter is defined in the direction along the length of the arm. It will be appreciated that the second gripping profile is intended to grip a needle such that the needle extends transversely i.e. perpendicular to the longitudinal axis of the forceps. Thus the width or diameter of the second gripping profile will correspond to the distance that the profile extends along length of the arm. Preferably the second gripping profile extends a distance along the length of the arm of no greater than 2 mm, or preferably no greater than 1.5 mm. Preferably the second gripping profile extends a distance along the arm of at least 0.1 mm. Preferably the second gripping profile extends a distance along the arm in the range of from 0.1 mm to 2 mm, and preferably in the range of from 0.1 mm to 1.5 mm.

Preferably the second gripping profile has a depth of from 0.05 mm to 2 mm, preferably from 0.1 mm to 1 mm, and in exemplary embodiments of around 0.5 mm. The width of the gripping profile may be different at the mouth to that at a depth within the profile. For example, the gripping profile may taper with depth. Thus, the second gripping profile may define a greater width/diameter at its mouth than at its deepest point, and may define a maximum width/diameter and, in embodiments, a minimum width/diameter. Where the width/diameter of the profile varies, the width/diameter at the mouth is preferably in the above ranges. This may be referred to as the width/diameter at the front of the gripping profile. The width/diameter at the rear of the second gripping profile i.e. within its depth may be less, and the gripping profile may taper to a point e.g. if it is of a triangular cross section, so as to be of negligible width at its rear. Thus the width/diameter at the rear of the profile may be at least 0.001 mm, at least 0.05 mm, or no greater than 2 mm, and in embodiments may be in the range of from 0.001 mm to 2 mm. In some embodiments, the minimum width/diameter may be around 0.5 mm.

The second gripping profile may be rectangular, square, triangular, V-shaped, hexagonal, elliptical etc in shape. These are the shapes defined in a vertical cross section i.e. taken parallel to the longitudinal axis of the arm, and are the shape defined by the space within the profile. Preferably the second gripping profile is arcuate, and most preferably defines an arc. In these embodiments the second gripping profile may be semi-circular.

Preferably the second gripping profile defines a channel for receiving the needle. The channel may have a width/diameter and/or depth in the above ranges, and may define maximum and minimum widths/diameters.

Preferably the second gripping profile is in the form of a groove. Preferably the second gripping profile includes only a single groove. The second gripping profile comprise a notch, which may be V-shaped. The maximum width or diameter of the second gripping profile will then be at the mouth of the profile.

The second gripping profile may define dimensions in any combination of the ranges described above.

It will be appreciated that the forceps of the present invention effectively include first and second gripping profiles configured to respectively snugly fit around the outer surfaces of objects of greater and less diameter to enable them to be securely gripped by the forceps when the gripping areas of the arms are brought together in use. Preferably the first gripping profile is arranged to fit around the outer surface of objects having an external diameter or width at least 10 times, or 15 times or 20 times or 30 times or 50 times greater than the diameter or width of an object around which the second gripping profile is arranged to fit. Preferably the first gripping profile extends a distance along the length of the arm at least 10 times, or at least 15 times, 20 times, 30 times or 50 times greater than the distance the second gripping profile extends along the length of the arm. Preferably, the first and second gripping profiles define respective first and second channels, the first channel having a width at least 10 times greater than the width of the second channel, or at least 15 times, 20 times, 30 times or 50 times greater. The relative widths are measured with respect to the greatest widths of the channels, i.e. at their respective mouths i.e. at the front of the profiles. Preferably the first channel has a depth at least twice as great as that of the second channel, or at least 5 times as great, or at least 10 times as great. Preferably the channels are separated from one another along the length of the arm by less than 4 mm, and preferably less than 2 mm. Preferably the first gripping profile is directly inboard of a proximal end of the first gripping profile. The channels may have any or all of the above relative dimensions.

The second gripping profile preferably defines a ratio of curvature which is at least 10 times greater than a ratio of curvature defined by the first gripping profile.

The first and second gripping profiles extend across at least a part of the width of the arm (the width being in the direction perpendicular to the longitudinal direction of the arms). Thus the gripping profiles define a dimension extending transverse to the longitudinal axis of the forceps i.e. parallel to the length of a syringe or needle when held in the forceps. The gripping profiles may extend over any portion of the width of the arm to render them capable of securely gripping needles or syringes in accordance with the invention. However, in preferred embodiments the first and/or second gripping profiles extend over the entire width of the arm, and preferably do so over their entire length. Thus the first and/or second gripping profiles preferably extend over the entire width of the arm i.e. across the arm from one side to the other. This will enable more secure gripping of a syringe, as the gripping profile will engage the outer surface of the syringe or needle over a greater portion of its length.

The first and/or second gripping profiles are preferably each symmetrical about a respective central axis extending in the direction parallel to the width of the arm, and/or parallel to the longitudinal axis of the arm.

In particularly preferred embodiments the gripping area of each arm comprises respective first and second gripping profiles of the construction described in respect of any of the above embodiments. The first and second gripping profiles of each of the arms are preferably of the same construction. Preferably, therefore, the gripping areas of each arm are matching gripping areas, defining matching first and second gripping profiles. In these particularly preferred embodiments, the gripping profiles of each of the opposed gripping areas are mirror images of one another about a longitudinal axis of the forceps.

It is envisaged that the first and second gripping profiles could at least partially overlap, provided that they still provide two distinct and different gripping profiles, and the arrangement does not interfere with independent operation of the first or the second gripping surfaces to grip either needles or syringes. For example, the second gripping profile might be formed in a part of the first gripping profile i.e. a groove formed in the arcuate surface of the first gripping profile. However, preferably the first and second gripping profiles are provided in different regions of the gripping area, and preferably are located adjacent one another.

It will be appreciated that forceps of the invention comprise a pair of arms have free distal ends which may be manipulated towards one another in use to grip objects between the respective gripping areas of the arms. As described above, the first and second gripping areas, and hence the first and second gripping profiles are located at the free ends of the arms.

The arms are connected to one another in a manner which allows them to be manipulated towards one another in use. Thus, the arms are preferably hingeably connected to one another. The arms are preferably integrally formed with one another. The hinge is therefore preferably integrally formed with the arms. This may be achieved as known in the art using appropriate variation in thickness of material etc.

Preferably the forceps are biased to automatically return to an open position in the absence of an opposing force.

Preferably the arms are connected to one another at their proximal ends i.e. the ends opposite to the distal ends which are manipulable towards one another for gripping objects. The arms may be connected to one another along a portion of their length extending away from the proximal ends, or only at the proximal ends, provided they may be manipulated towards one another in use. In these preferred embodiments, the arms define jaws, and may be operated in the manner of tweezers.

Each arm of the forceps defines an intermediate portion between its proximal and distal ends.

The forceps are preferably of the type intended to be operated manually by a user by applying a force acting to urge the gripping areas together. The forceps are preferably hand held forceps, which may be operated in one hand. The force urging the arms together is applied in an intermediate region of the forceps between the connected and free ends of the arms in preferred embodiments. Preferably the forceps comprise an operating region located between the proximal and distal ends of the arms i.e. in the intermediate region. The operating region is a region intended to be manually gripped by a user. The operating region is preferably located at a distance of at least a quarter, and preferably at least a third of the length of the forceps from the distal end of the forceps i.e. the distal ends of the arms, and preferably the operating region is located in the central third of the length of the forceps. This may reduce the likelihood of a user inadvertently touching a sharp if their hand slips.

The operating region may be an area which is in some way marked to denote to a user that this is the area they should grip, e.g. by colour, some formation etc. The operating means may comprise manual locating means to facilitate locating of the user's hand appropriately. The manual locating means may comprise any suitable means associated with the outer surface of the or each arm. The manual locating means may comprise one or more formations in the outer surface of the or each arm, e.g. a groove, or may be defined by the absence of a formation extending on either side of the intended operating region. Preferably the operating region comprises means for facilitating manual gripping by a user. The means for facilitating gripping by a user preferably comprises a portion of the outer surface of the or each arm which is configured to facilitate manual gripping relative to the adjacent parts of the arm. In preferred embodiments the means for facilitating manual gripping comprises a region having a greater coefficient of friction than adjacent regions along the length of the, outer surface of the or each arm. The means for facilitating gripping should resist downward slippage of the user's hand towards the free ends of the arms. The means for facilitating gripping may comprise a region of the or each arm which has been treated or formed in a suitable manner. In embodiments the region comprises suitable formations, e.g. corrugations, grooves, scoring etc. for this purpose, or may be a region which has been treated with a substance to increase its frictional resistance. Preferably the means for facilitating gripping comprises a plurality of grooves in the outer surface of the or each arm. The operating region may be defined by the region having the means for facilitating gripping.

Preferably the or each arm comprises guard means to inhibit sliding of a user's hand into the proximity of the gripping area of the arm. The guard means may be arranged to prevent or inhibit sliding of the user's hand to closer than a given distance from the distal end of the arm. Thus, the guard means prevents a user's hand sliding downward into the proximity of the gripping areas of the forceps in use. Preferably the guard means comprises a projection extending outwardly from the outer surface of the or each arm.

The guard means is located between the operating region and the gripping area of the arm. Preferably the guard means is located at the distal end of the operating region. The guard means may then define the distal end of the operating region. Thus the guard means is preferably located at the distal end of the means for facilitating manual gripping.

Preferably the guard means is located a distance from the distal end of the or each arm a distance corresponding to the distance of the operating region from the send of the arm. Thus, preferably the guard means is located at a distance of at least 25%, and preferably at least 33% of the length of the arm from the distal end of the arm, and preferably between 33% and 66% of the length of the arm from the distal end.

Preferably the or each arm comprises a distal connecting portion extending between a proximal end of the gripping area and the distal end of the operating region. Preferably the distal connecting portion has a length of at least 25 %, preferably at least 100%, and more preferably at least 150% or 200% of a length of the gripping area. In preferred embodiments where guard means is located at the distal end of the operating region, the distal connecting portion extends between a proximal end of the gripping area and the guard means. Preferably the distal connecting portion tapers towards the distal end. In embodiments, the distal portion defines a neck above the gripping area.

Preferably each arm comprises a proximal connecting region extending between a proximal end of the operating region and the proximal end of the arm.

Preferably the arms diverge with one another in the direction from their proximal ends towards the operating region. The arms may converge with one another in the direction from the operating region towards their distal ends. The arms may then define a wishbone type shape.

The guard means may be located in the region of greatest separation of the arms.

The gripping areas of each arm are those areas intended to engage with objects to be gripped in use. The gripping areas may be inclined towards one another. In embodiments an or each arm comprises an enlarged head portion at its distal end which comprises the gripping area. The head portion is enlarged at least relative to the adjacent region of the arm towards its proximal end, e.g. relative to a distal connecting region extending between the gripping area and the operating region in embodiments.

Preferably an or each arm terminates in a paddle at its distal end, and the gripping area is provided on the inner surface of the paddle. In these embodiments, a distal connecting region of the arm may extend between the distal end of the operating region and the proximal end of the paddle. The connecting region may taper towards the proximal end of the paddle. Thus the paddle preferably provides an enlarged head portion at the end of the arm. Preferably the paddle is square or rectangular in shape.

Preferably the or each arm is blunt ended at the distal end. Preferably the or each arm is rectangular or square ended at its distal end. Preferably the gripping areas of the arms do not taper. This may facilitate picking up of needles in contrast to some conventional forceps having pointed ends. In embodiments in which the arm terminates in a paddle at its distal end, preferably the or a paddle where provided, is straight ended, and preferably rectangular or square ended.

It will be appreciated that, particularly when the forceps are formed from a more flexible material, the arms may tend to move laterally with respect to one another as they approach one another if the user does not apply a force precisely in the correct direction. This is undesirable, as it may lead to the forceps skidding over objects, increasing risk of contamination of the sharps to be collected, and increasing the risk to the user. Preferably the forceps comprise guide means to guide the arms as they move towards one another to grip an object, at least over a final part of the movement of the arms toward one another. Preferably the guide means is configured to inhibit lateral movement of the arms with respect to one another. It will be appreciated that lateral movement is movement in the cross direction relative to the main direction in which the gripping areas move in order to grip an object, and which may cause the gripping surfaces to shear over one another.

Preferably the guide means is arranged to maintain the arms spaced from one another unless the gripping areas of the arms are laterally aligned. Thus, the guide means may comprise cooperating formations on the opposing surfaces of the respective arms which are arranged to allow the arms to be brought closer together only when the gripping areas are aligned with one another. The cooperating formations may engage one another when the gripping areas are not laterally aligned with one another to prevent further movement of the gripping areas towards one another. This may be achieved using cooperating formations on the respective arms which are offset from one another, or by providing formations which mate with one another when the gripping areas are aligned to allow the gripping areas to be brought closer together. The guide means may maintain the arms spaced from one another to a degree which prevents them from being operable to grip a needle or syringe when not properly aligned.

The guide means may be of any suitable form. In preferred embodiments the guide means comprises guide teeth. In embodiments opposed teeth are provided on the inner surfaces of the arms for this purpose. There may be one or more teeth on each arm. The respective arms may comprise opposing teeth which are laterally offset from one another. In this way the teeth may allow the arms to be moved close to one another when the teeth are laterally aligned, but may engage one another to maintain the arms separated from one another when they are not laterally aligned. In embodiments, one of the arms may comprise a plurality of teeth arranged to receive an opposed tooth of the other arm therebetween when the arms are aligned with one another. Preferably the guide means is located on the interior surface of the or each arm in the operating region.

The forceps may be of any suitable dimension, depending upon the intended use. The forceps are intended to be operable using a single hand. The gripping areas should be of suitable dimensions to accommodate the first and second gripping profiles. Preferably the width of each gripping area is in the range of from 2 mm to 50 mm. The width of the gripping area is measured perpendicular to the length of the arm. Preferably, the gripping areas extend a distance in the range of from 5 mm to 2.5mm along the length of each arm from its distal end. In embodiments in which the gripping areas are provided on paddles at the end of each arm, preferably the paddles have a width and length in these ranges. Preferably the length of the forceps is in the range of from 50 mm to 300 mm.

Preferably the forceps are single piece forceps. Thus all of the parts of the forceps are integrally formed, and the first and second gripping profiles are integrally formed with the remainder of the forceps. The forceps are disposable forceps i.e. intended for use on a single occasion, and not intended to be cleaned for reuse. The forceps are plastic forceps. For example, the forceps may be formed of a polymeric material e.g. polypropylene. In particularly preferred embodiments the forceps are formed by an injection moulding technique. The material of the forceps may be selected as desired for a given application. In embodiments, the forceps may be constructed such that the arms exhibit some flexibility, at least towards the free ends. This may facilitate manipulation of the forceps to pick up sharps.

Preferably the first and second gripping profiles are the only gripping profiles provided on the gripping areas, and preferably the only gripping profiles present on the inner surfaces of the arms, at least below the operating region. In embodiments, the proximal end of the first gripping profile defines the proximal end of the gripping area.

Thus the features described above in respect to the arms may apply to one, or more preferably both arms. Thus references to the arm, should encompass an arm or each arm. It will be appreciated that preferably each arm of the forceps is of the same construction. Thus each arm preferably comprises at least gripping areas of the same construction i.e. including matched first and second gripping profiles. Preferably each arm includes identical features in other respects, including for example, guide means, guard means, operating regions etc. In preferred embodiments, the forceps are symmetrical about a longitudinal axis.

It has been found that the forceps are particularly advantageous in the context of disposing of sharps, and may be used to independently pick up needles or syringes as required to place them in a container for disposal, or transport to an analysis or sample retaining centre etc.

Further, a kit comprising forceps in accordance with any of its embodiments, and a container configured for receiving syringes retrieved using the forceps, is disclosed.

The container may be of any configuration provided that it may house syringes of the size expected to be retrieved, and while it is preferably cylindrical, may be of other shapes, such as oval, square, rectangular or triangular in cross-section. The container must have dimensions sufficient to accommodate the largest dimensions of the syringes. In particular, the container should have a greatest diameter or width sufficient to accommodate the shoulders of a syringe. The length of the container may be in the range of from 50 mm to 300 mm. The greatest diameter or width of the container may be in the range of from 5 mm to 50 mm. The greatest width of the container is preferably in the region of its open end. The open end of the container is at a proximal end thereof. The container may taper along its length towards a distal end.

The container may be of any suitable form, and should be of dimensions which enables it to house syringes. The container is an elongate container. It will be appreciated that if it is of sufficient dimension to accommodate syringes, the container will also be suitable for receiving smaller dimension needles, and its dimensions should therefore be chosen with respect to syringes, which are the limiting factor.

The container should be a closable container, and is preferably a sealable container. Preferably the container comprises a cover which may be closed over an open end of the container. Preferably the cover is in the form of a lid, and is preferably attached to the container, preferably by a strap. This may facilitate one handed operation to close the container. In these embodiments, the container thus defines a main body having an open end for receiving syringes and a cover. The main body of the container is preferably of the dimensions discussed above. The cover may be arranged to lockingly engage the main body of the container. In preferred embodiments the cover comprises a plurality of locking lugs arranged to engage with a rim of the main body of the container to lock the cover to the main body. Preferably the cover is arranged to sealingly close the main body to seal syringes in the container.

In some embodiments, the container comprises means for providing a tamper evident safety seal sealing the container after syringes and/or needles have been located therein in use. Preferably the tamper evident safety seal is arranged to seal the cover or lid of the container to the container i.e. the main body thereof. The container and/or the cover may comprise such means. Thus, the main body and/or cover of the container may comprise a component or components which may be used by a user to provide a tamper evident safety seal between the main body of the container and cover. For example, the seal may extend downward from the lid of the container toward the base to provide a seal with the main body of the container. The seal may comprise a strap or straps. The tamper evident seal is arranged such that it may be applied by a user to the container after syringes and/or needles have been located therein. The tamper evident seal is preferably configured to provide an additional or secondary seal joining the cover/lid to the container, i.e. in addition to a seal obtained by locating the cover over an end of the container as described above. The tamper evident seal is configured to be visible from the exterior of the container to enable visually detection as to whether the container has been tampered with after use. Such seals may be referred to as tamper evident permanent fixed seals, as they may provide a permanent fix seal of the container.

In accordance with the invention in any of its embodiments, preferably the container is a rigid container.

Preferably the container is a plastic container.

The container is preferably a disposable container intended for single use before being discarded, and not to be cleaned for reuse.

The kit may further comprise one or more of; a wet-wipe, e.g. a disinfectant wipe, a disposal bag, and disposable gloves. The kit may be contained in an outer package, preferably a plastic bag. Further, a single use kit including all of the items necessary for a user to safely retrieve and store retrieved sharps is disclosed.

The present invention further extends to forceps in accordance with the invention as claimed in combination with a needle or a syringe e.g. held respectively in the second or first gripping profile.

A method of using the forceps is further disclosed; the method comprising manipulating the gripping areas of the arms toward one another such that the or each second gripping profile securely grips a needle. The method preferably extends to the step of depositing the needle and manipulating the gripping areas of the arms toward one another such that the or each first gripping profile securely grips a syringe. In other embodiments, the method comprise manipulating the gripping areas of the arms toward one another to securely grip a syringe using the or each first gripping profile of the gripping areas, and preferably depositing the syringe and then manipulating the gripping areas of the arms toward one another to grip a needle using the or each second gripping profile of the gripping areas.

The method may further comprise depositing the needle or syringe in a container and sealing the container.

Some further embodiments of the present invention will now be described by way of example only and with reference to the accompanying drawings of which:
Figure 1 is a view of forceps in accordance with one embodiment of the invention when in their open configuration and from one side;
Figure 2 is a sectional view along the longitudinal axis of the forceps of Figure 1 along the line A-A showing the arm to the left hand side of the view of Figure 1 from its interior;
Figure 3 is a view corresponding to that of Figure 2 but showing the outer surface of the arm;
Figure 4A is a schematic view from above of the paddle region at the end of the arm 6 illustrating some of the possible ranges of dimensions for the first and second gripping profiles in accordance with some preferred embodiments;
Figure 4B is a vertical cross sectional view of the paddle/gripping area of Figure 4A showing the first and second gripping profiles in more detail, and with a detail in the region of the second gripping profile;
   are respective schematic views of the forceps being used to pick up a syringe and a needle;
Figures 5A and 5B illustrate the use of the forceps to pick up a syringe and a needle respectively;
Figure 6A illustrates a closable container which may be used to receive syringes or needles picked up by the forceps, and which may be included in a kit together with the forceps;
Figure 6B is a view from the top of the container through its open end in the direction of arrow A in Figure 6 showing the configuration of the open end in more detail;
Figure 6C illustrates other examples of cross sectional shapes of the container which may be used in accordance with further embodiments of the invention; and
Figure 7 illustrates schematically insertion of a syringe into the container.

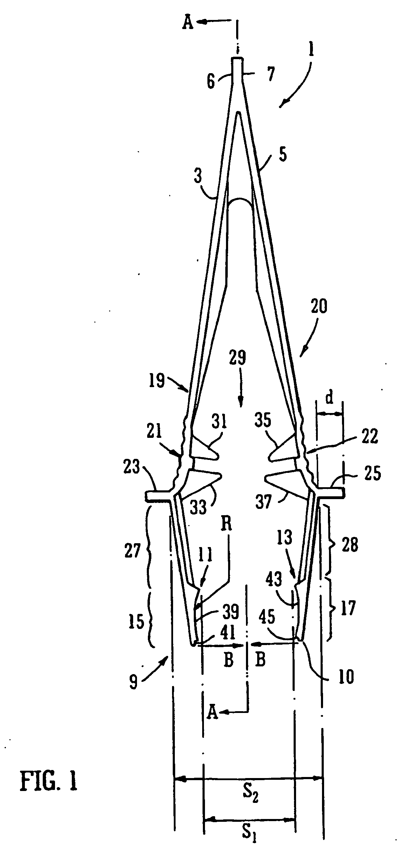

Forceps in accordance with one embodiment of the invention will now be described with respect to Figures 1 to 3. The forceps are plastic forceps 1, being integrally formed by an injection moulding technique. The forceps may be formed of polypropylene. The forceps define a longitudinal axis along line A-A of Figure 1, and it will be seen that the forceps are symmetrical about the longitudinal axis.

The forceps include a pair of arms 3, 5. The first and second arms 3 and 5 of the pair of arms are connected to one another at one of their proximal ends 6, 7. The other distal ends 9, 10 of each of the arms are free and may be manipulated towards one another for gripping syringes and needles in use. The distal ends 9, 10 of each of the arms define respective opposed gripping areas 11, 13 which face one another in use.

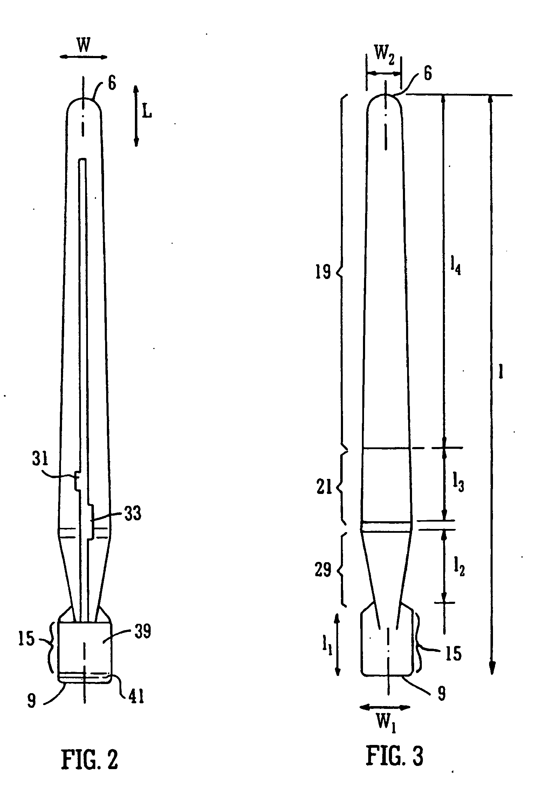

The gripping areas are provided on the interior surfaces of enlarged head portions in the shape of paddles 15, 17 located at the distal or gripping end of each arm. The paddles 15, 17 can be seen more clearly in Figures 2 and 3. Each paddle 15, 17 is rectangular in longitudinal cross-section, and does not taper. It is blunt ended. By providing paddles of this configuration, the gripping areas 11,13 and their associated gripping profiles also do not taper. This may facilitate gripping of syringes, in contrast to some conventional pointed ended forceps. The arms exhibit some degree of resilience, at least in the region towards their distal ends to facilitate gripping of objects.

Each arm defines an intermediate portion between its respective distal end 9, 10 and its proximal end 6, 7. Each arm comprises an operating portion located between its ends i.e. in the intermediate portion, which is intended to be gripped manually by a user in use to operate the forceps. The operating portion of each arm comprises a manual gripping area 21, 22 comprising formations in the form of a plurality of grooves in the outer surface of the arm to facilitate gripping. In this embodiment the manual gripping area defines the operating portion of the respective arm. The distal gripping ends 9, 10 of the forceps are moveable towards one another from the open position shown in Figure 1 in the direction of the arrows B of Figure 1 in order to grip syringes/needles when the user applies a force to the operating portion 21, 22 of the arms, urging the arms toward one another. In the absence of any opposing force, the arms are biased to return to the open position shown in Figure 1.

Each arm further comprises a guard 23, 25 extending outwardly from the outer surface of the arm 3, 5 to prevent a user's fingers sliding down from the operating region 21, 22 towards the distal end 9, 10 of the arm into the proximity of the syringe/needle gripping region 11, 13 of the arm. In this manner, the guard helps to prevent the user's fingers accidentally coming into contact with sharps which are to be picked up. The guard 23, 25 therefore defines a distal end of the operating portion 21, 22 and the grooves of the manual gripping portion terminate at the guard 23, 25. In the illustrated embodiment, the arms 3, 5 are inclined towards one another in the region below the operating portions 21, 22 in the direction towards their distal ends 9, 10. The arms 3, 5 are inclined towards one another in the region above the operating portions 21, 22 in the direction towards their proximal ends 6, 7. The arms therefore define a greatest degree of separation in the absence of any force urging the arms together in the region of the distal end of the operating portions 21, 22, i.e. in the region of the guards 23, 25.

Each arm defines a respective distal connecting portion 27, 28 extending between a proximal end of the gripping area 11, 13 of the respective arm, and the distal end of the operating portion 21, 22 of each arm. Each arm defines a proximal connecting portion 19, 20 extending between the proximal end of the operating portion 21, 22 of each arm and the proximal end 6, 7 of the arm.

The forceps further comprises guide means 29 comprising respective pairs of opposed guide teeth 31, 33, 35 and 37 projecting towards one another from the inner surfaces of each of the arms 3,5. The guide means 29 is intended to inhibit lateral movement between the distal ends 9, 10 of the arms as they approach one another, at least over the final part of their movement towards one another. In this way the guide means 29 guides the arms laterally so that they can be brought towards one another in a manner such that the gripping areas 11, 13 are aligned with one another. This prevents skidding or sliding of the distal ends 9, 10 and gripping areas 11, 13 relative to one another. If the arms are not correctly laterally aligned with one another, the teeth engage one another preventing movement of the distal ends of the arms 3, 5 and hence the gripping areas 11, 13 closer to one another, thereby maintaining the arms 3, 5 spaced from each other by an amount which inhibits operation to grip needles/syringes.

Although not clearly shown in Figure 1, the guide teeth 31, 33, 35, 37 operate in this manner because the respective cooperating pairs of teeth 33, 37 and 31, 35 on each arm 3, 5 are laterally offset from one another. The positions of the teeth on one of the arms 3 is schematically shown in Figure 2. It will be appreciated that the guide means may be provided in another form. For example, there may be a different number of teeth on one or both of the arms. In some embodiments, there may be a single tooth on one of the arms 3, 5 and a plurality of teeth, preferably a pair of teeth on the other one of the arms. For example, there may be a single tooth on one arm and a pair of teeth on the opposite arm, The pair of teeth may define a space therebetween for receiving the single tooth of the other arm, such that the teeth will act to space the arms from one another unless they are aligned such that the single tooth enters the space defined between the two teeth on the opposing arm.

In accordance with the invention, the gripping area 11, 13 of each arm is provided with two different gripping profiles. The first gripping profiles 39, 43 are adapted for engaging and gripping syringes. Each arm comprises a second gripping profile 41, 45 which is located at the distal tip of the arm. The second gripping profile is configured to snugly fit around a needle to enable gripping of needles between the arms. The first gripping profile 39, 43 of each arm is located directly inboard of the second gripping profile.

To this end the first gripping profiles 39, 43 have a significantly lesser radius of curvature than the profiles 41, 45 for picking up needles. The first gripping profiles 39, 43 are arcuate in order to snugly fit around the barrel of a syringe. The first gripping profiles 39, 43 are of dimensions which enable them to fit around the outer surface of objects having a diameter in the range of typical syringe barrels. The first gripping profiles 39, 43 define substantially smooth syringe engaging surfaces.

The second gripping profiles 41, 45 are in the form of indentations or grooves in the respective gripping areas 11, 13 of each arm. In the embodiments illustrated, the second gripping profiles are in the form of notches in the gripping areas 41, 45 adjacent the distal tips of the arms. The second gripping profiles 41, 45 are arranged for picking up needles and thus the second gripping profiles are of dimensions suitable to fit around the outer surface of objects having the diameter of a needle.

The first and second gripping profiles may be seen as defining respective channels of greater and lesser width respectively. The width of the channels is measured in the direction parallel to the longitudinal axis of the arms.

It will be seen that in the embodiments illustrated, the first and second arms 3, 5 have matching first and second gripping profiles 39, 43 and 41, 45.

As described above, the actual dimensions of the first and second gripping profiles 39, 43 and 41, 45 will vary depending upon the range of size of syringe and/or needles to be picked up. Similarly, the dimensions of the overall forceps will vary. As shown in Figure 2, the forceps define a width in the direction W, which may be referred to as the lateral direction, and a length L in the longitudinal direction. By way of example, as shown in Figure 3, the overall length 1 of the forceps between the distal end and proximal end of the forceps, corresponding to the length of each of the arms, may be in the range of from 50 mm to 300 mm. The width w₁ of the gripping area 11, 13 of each arm having the first and second gripping profiles, which corresponds to the dimensions of the paddle region 15, 17 in the illustrated embodiments, may be in the range of from 2 mm to 50 mm. The first and second gripping profiles extend over the entire width of the gripping area of each arm and therefore may have a dimension in the direction of the width of the arm in the above ranges. Figures 2 and 3 show the forceps from the left hand side of Figure 1, therefore only the arm 3 is visible. The construction of the other arm 5 is identical.

With reference to Figure 3, in one embodiment, the length 1 of the forceps was 130 mm and the width w₁ of each arm in the region of the gripping area 11/paddle 15 (the inner surface of the paddle defines the gripping area in these embodiments) was 13 mm. The width w₂ of the arms at the opposite proximal ends was 8 mm. Each gripping area 11 had a length I₁ of 15 mm, and the distal connecting portion 27 extending between the distal end of the operating portion 21 and the proximal end of the paddle/gripping area 15 had a length I₂ of 24 mm. The operating portion 21 having the grooved manual gripping surface had a length L₃ of 15 mm. The proximal connecting portion 19 extending between the proximal end of the operating portion 21 and the proximal end 6 of the arm has a length I₄ of 76 mm.

In the arrangement shown in Figure 1, the forceps had a natural open position in the absence of any force urging the arms together in which the distal ends 9, 10 of the arms were separated by a distance s₁ of 30 mm, corresponding to a maximum separation of the arms at the distal end of the operating portions 21, 22 in the region of guard 23, 25 of 40 mm (s₂). In the embodiment illustrated the guards 23, 25 projected outwardly a distance (d) of 4 mm from the manual gripping/operating portion 22 in the plane parallel to the direction of operation of the forceps. The first gripping profile 39 defined a radius of curvature of 11 mm, and thus the arcuate first gripping profile subtended an angle of about 50 degrees.

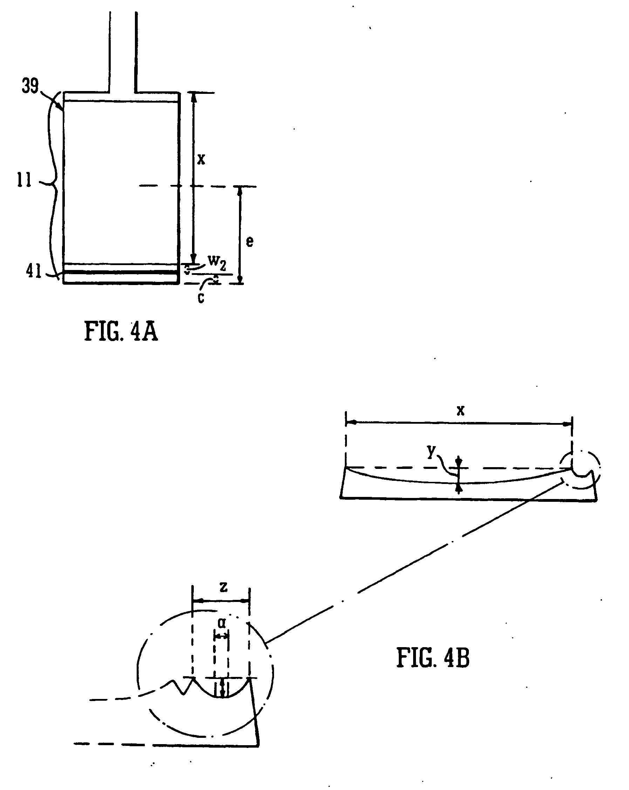

The preferred dimensions of the first and second gripping profiles will now be described with respect to one exemplary embodiment, with reference to Figures 4 A and B. The first gripping profile 39 is arranged to grip syringes having an external diameter in the barrel region of between 5 mm and 20 mm. The first syringe gripping profile 39 is arcuate and defines a channel having a width x in the range of from 3 mm to 20 mm, in one example being 13 mm. This corresponds to the distance the first gripping profile extends along the length of the gripping area 11. The width is measured at the mouth of the channel. The first gripping profile 39 extends across the entire width of the paddle like end of the arm i.e. from one longitudinally extending side to the other. The channel has a greatest depth y in the range of from 0.5 mm and 5 mm, and in the example the depth is 2.5mm.

The second gripping profile 41 is adapted to grip needles having an external diameter in the range of from 0.1 mm and 1.5 mm. The second needle gripping profile 41 defines a channel having a maximum width z at its front/mouth in the range of from 0.1 mm and 2 mm, and the example the width is 1.2 mm. The channel defines a minimum width a at its rear a of in the range of 0.01 mm to 1 mm, and in the example, the width is 0.01 mm. The channel has a depth b in the range of from 0.05 mm and 2 mm, and in the example the depth is 0.5 mm.

The first gripping profile 39 has a centre which is located at a distance e in the range of from 2.4 mm to 12.8 mm from the distal end of the paddle/arm. In the example the distance e is 8.5 nun. The second gripping profile 41 has a centre which is located at a distance c of 0.15 mm to 1.1 mm from the distal end of the arm, and in the example at a distance c of 0.7mm.

The first and second gripping profiles may each be e.g. square, rectangular, hexagonal, triangular, elliptical, arcuate or semi-circular. In the example, each is arcuate.

In the embodiment, the first gripping profile has a width at least 10 times as great as the width of the second gripping profile.

The other arm 5 and its gripping area and profiles is of the same dimensions.

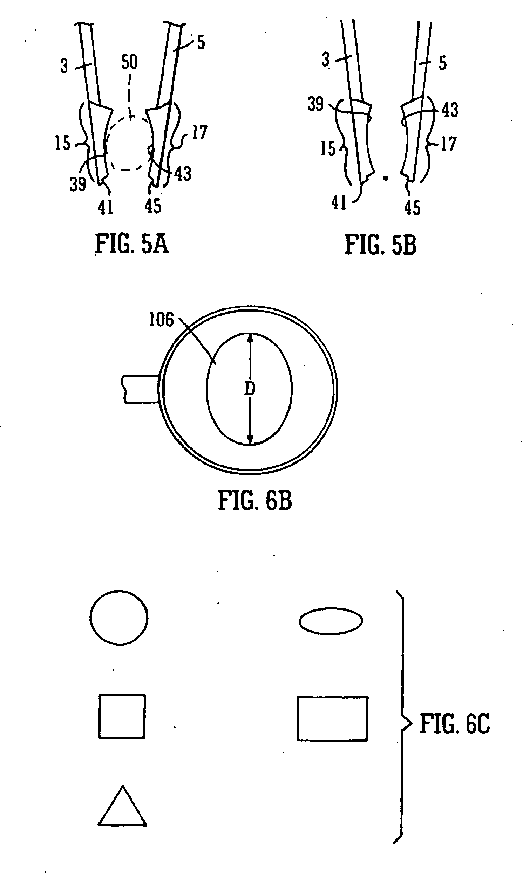

Figures 5A and 5B illustrate schematically the operation of the forceps to pick up either a syringe (Figure 5A) or a needle (Figure 5B).

In the first case, the syringe barrel 50 is located between the matched first gripping profiles 39 and 43 provided on the gripping areas 11, 13 on the inner surfaces of the paddles 15, 17 at the distal ends of each arm 3, 5. The user then manually urges the distal ends of the arms towards one another by exerting a force on the manual gripping regions 21, 22 of the operating portions of each arm. In doing this, the guards 23, 25 prevent the user's fingers from sliding down from the grooved gripping regions 21, 22 closer to the distal ends 9,10 of the arms, reducing the risk that the user might touch a needle or syringe. The guide teeth 29 act to prevent lateral movement between the gripping areas/distal ends of the arms as they are manipulated towards one another. By manipulating the distal ends towards one another, the opposed first gripping profiles 41, 45 snugly engage around opposite sides of the barrel of the syringe, allowing the syringe to be picked up in a similar manner to that when using tweezers.

Operation to pick up a needle proceeds effectively in the same manner.
However, rather than locating the first gripping profiles 39, 43 around the needle, instead the second gripping profiles 41, 45 at the distal tips of the respective arms 3, 5 are located on either side of the needle, such that the needle is snugly held between the second gripping profiles on each arm when the arms are urged together.

It will be appreciated that because the profile for picking up needles is located at the distal end of each arm of the forceps, the forceps may be more readily used to pick up needles using a simple up and down movement without needing to try to scoop up the needle, or slide the ends of the arms underneath the needle. When it is instead attempted to pick up a syringe, the second needle gripping profile does not interfere with the operation of the first gripping profile, as it is located at the distal tip of the forceps, with the first gripping profile being located directly inboard of the second gripping profile. Similar ease of operation is offered, allowing syringes to be picked up with a simple up and down pincer-like movement.

The forceps are particularly suited for use in conjunction with a sharps disposal system. Increasingly, it is necessary to retrieve sharps e.g. discarded needles and/or syringes and place them in a container, e.g. for disposal, retention, or analysis purposes.

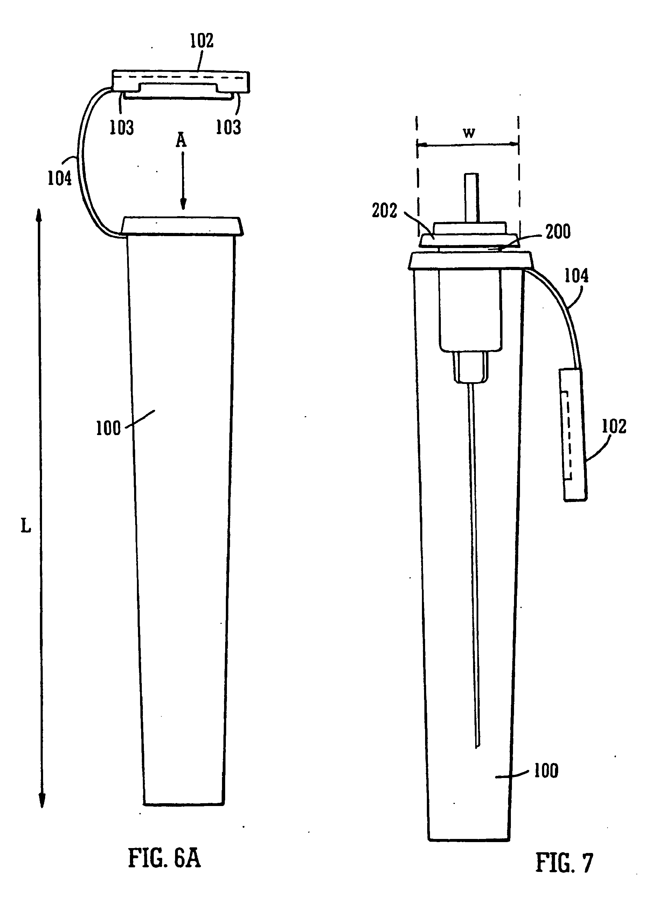

One embodiment of a suitable sharps container is shown in Figures 6A and 6B. Figure 6B is a view from above in direction of the arrow A in Figure 6A showing the open top of the container in more detail. Figure 7 schematically illustrates insertion of a syringe in the container.

The container is a plastic, elongate container 100 which has an open end 106. A lid 102 is connected to the container in the region of the open end by a strap 104. In use a syringe held in the forceps is dropped into the open top 106 of the container, and the lid 102 closed over the mouth of the container to secure and seal the syringe in the container for retention, transport or other processing e.g. for disposal, evidence or analysis. The lid comprises lugs 103 which cooperate with the rim of the main body of the container 100 to lock the lid thereto. In the embodiment illustrated, the main body of the container 100 tapers in the direction away from its open end towards its base. This is to accommodate a typically shaped syringe in which the widest portion of the syringe is located towards its proximal end, i.e. further from the needle, in the region of a shoulder portion of the syringe located e.g. between the plunger and the barrel. This is illustrated schematically in Figure 7, which shows a syringe 200 being inserted into the container 100. The shoulders 202 of the syringe 200 have a width w which is less than the maximum diameter D of the opening 106 at the end of the container 100. Of course, the container should remain of a dimension greater than that of the shoulders or maximum width of the syringe below the opening for a distance sufficient to accommodate the syringe.

The opening 106 is being shown as being approximately oval in cross section (as viewed in horizontal cross section) in Figures 6B. However, it will be appreciated that the cross section of the opening, and indeed of the container as a whole, may be chosen to be of any shape provided that they sallow a syringe to be inserted into the container. For example, the opening may be square, rectangular, triangular or circular in horizontal cross section. In preferred embodiments the container is of the same shape. Other examples of cross sectional shapes are given in Figure 6C.

The overall length of the container may be in the region of from 50 mm to 300 mm. This has been found suitable to accommodate a wide range of different sized syringes. The greatest diameter or width D of the opening of the container may be in range of from 5 mm to 50 mm. This has been found to permit insertion of syringes of most typical encountered dimensions into the container.

The container may also include means for providing a tamper evident seal between the lid and the main body of the container after the sharps have been located in the container, and the lid locked to the main body in use as described above. This is not shown in the drawings. The main body and/or lid may comprise components which may be associated with one another to provide such a seal. For example, the lid may include a component extending downwardly therefrom which may be used to provide a permanent fix seal with the main body. The tamper evident seal provides a secondary seal which may allow someone to visually detect whether the container has been tampered with after initially being closed.

## Claims

1. Forceps;
wherein the forceps comprise a pair of arms (3,5), the arms defining respective opposed gripping areas (11, 13) at their distal ends (9,10), the gripping areas (11, 13) being manipulable towards one another in use for gripping objects;
wherein at least one of said gripping areas (11, 13) comprise:
a first gripping profile (39,43) wherein the first gripping profile (39,43) is for gripping syringes, and is adapted to snugly fit around the barrel of a syringe, wherein the first gripping profile (39,43) is arcuate and the forceps are disposable plastic forceps, and **characterised in that,** , the at least one of said gripping areas (11,13) further comprises a different second gripping profile (41,45)in the form of a notch extending across the arm, wherein the second gripping profile is for gripping needles, and is configured to snugly fit around a needle in the form of a notch extending across the arm, and wherein the second gripping profile is located between the first gripping profile and a free distal end of the arm.

2. The forceps of claim 1, wherein the second gripping profile (41,45) is located at the distal tip of the arm.

3. The forceps of any preceding claim, wherein the first gripping profile (39,43) is located directly inboard of the second gripping profile.

4. The forceps of any preceding claim, wherein the first gripping profile (39,43) is adapted to snugly fit around the outer surface of an object having a width or diameter in the range of from 5 mm to 20 mm, and/or wherein the second gripping profile (41,45) is adapted to snugly fit around the outer surface of an object having a width or diameter in the range of from 0.1 mm to 1.5 mm.

5. The forceps of any preceding claim, wherein the first gripping profile (39,43) defines a channel in the gripping area having a width in the range of from 3 mm to 20 mm, and/or wherein the second gripping profile (41,45) defines a channel in the gripping area having a width in the range of from 0.1 mm to 2 mm.

6. The forceps of any preceding claim, wherein the first gipping profile (39,43) has a depth in the range of from 0.5 mm to 5 mm, and/or wherein the second gripping profile (41,45) has a depth in the range of from 0.05 mm to 2 mm.

7. The forceps of any preceding claim, wherein the gripping areas (11,13) of the arms comprise matched first and second gripping profiles.

8. The forceps of any preceding claim, wherein each arm (3,5) comprises an operating region comprising means to facilitate manual gripping of the forceps by a user, and wherein the forceps further comprise guard means (23,25) located between the operating region and the gripping area, preferably wherein the forceps further comprise a distal connecting portion extending between the guard means and the gripping area of the arm.

9. The forceps of any preceding claim, further comprising guide means (31,33,35,37) to guide the arms as they move towards one another, wherein the guide means comprises guide teeth.

10. The forceps of any preceding claim wherein the forceps have a length in the range of from 50 mm to 300 mm, and/or wherein each arm defines a width in the region of the gripping area in the range of from 2 mm to 50 mm.

## Patentansprüche

1. Zange,
wobei die Zange ein Paar von Armen (3, 5) aufweist, wobei die Arme jeweils gegenüberliegende Greifflächen (11, 13) an ihren distalen Enden (9, 10) definieren, wobei die Greifflächen (11, 13) bei Gebrauch zum Ergreifen von Objekten zueinander manipulierbar sind;
wobei zumindest eine der Greifflächen (11, 13) Folgendes aufweist:
ein erstes Greifprofil (39, 43), wobei das erste Greifprofil (39, 43) zum Ergreifen von Spritzen dient und so ausgebildet ist, dass es fest um die Trommel einer Spritze sitzt, wobei das erste Greifprofil (39, 43) bogenförmig ist und die Zange eine Kunststoffzange für den Einmalgebrauch ist, und **dadurch gekennzeichnet, dass** die zumindest eine der Greifflächen (11, 13) des Weiteren ein anderes zweites Greifprofil (41, 45) in der Form einer Kerbe aufweist, die sich über den Arm erstreckt, wobei das zweite Greifprofil zum Ergreifen von Nadeln dient und so ausgebildet ist, dass es fest um eine Nadel sitzt, und wobei das zweite Greifprofil zwischen dem ersten Greifprofil und einem freien distalen Ende des Arms positioniert ist.

2. Zange nach Anspruch 1, wobei das zweite Greifprofil (41, 45) an der distalen Spitze des Arms positioniert ist.

3. Zange nach einem der vorangehenden Ansprüche, wobei das erste Greifprofil (39, 43) direkt nach innen gerichtet von dem zweiten Greifprofil positioniert ist.

4. Zange nach einem der vorangehenden Ansprüche, wobei das erste Greifprofil (39, 43) dazu ausgebildet ist, fest um die Außenfläche eines Objekts mit einer Breite oder einem Durchmesser im Bereich von 5 mm bis 20 mm zu sitzen und/oder wobei das zweite Greifprofil (41, 45) dazu ausgebildet ist, fest um die Außenfläche eines Objekts mit einer Breite oder einem Durchmesser im Bereich von 0,1 mm bis 1,5 mm zu sitzen.

5. Zange nach einem der vorangehenden Ansprüche, wobei das erste Greifprofil (39, 43) einen Kanal in der Greiffläche mit einer Breite von 3 mm bis 20 mm definiert und/oder wobei das zweite Greifprofil (41, 45) einen Kanal in der Greiffläche mit einer Breite im Bereich von 0,1 mm bis 2 mm definiert.

6. Zange nach einem der vorangehenden Ansprüche, wobei das erste Greifprofil (39, 43) eine Tiefe im Bereich von 0,5 mm bis 5 mm hat und/oder wobei das zweite Greifprofil (41, 45) eine Tiefe im Bereich von 0,05 mm bis 2 mm hat.

7. Zange nach einem der vorangehenden Ansprüche, wobei die Greifflächen (11, 13) der Arme zusammenpassende erste und zweite Greifprofile aufweisen.

8. Zange nach einem der vorangehenden Ansprüche, wobei jeder Arm (3, 5) eine Bedienungsregion aufweist, die Mittel zum Erleichtern des manuellen Ergreifens der Zange durch einen Benutzer aufweist, und wobei die Zange des Weiteren Schutzmittel (23, 25) aufweist, die zwischen der Bedienungsregion und der Greiffläche positioniert sind, wobei vorzugsweise die Zange des Weiteren einen distalen Verbindungsabschnitt aufweist, der sich zwischen dem Schutzmittel und der Greiffläche des Arms erstreckt.

9. Zange nach einem der vorangehenden Ansprüche, des Weiteren aufweisend Führungsmittel (31, 33, 35, 37) zum Führen der Arme, während sie sich zueinander bewegen, wobei das Führungsmittel Führungszähne aufweist.

10. Zange nach einem der vorangehenden Ansprüche, wobei die Zange eine Länge im Bereich von 50 mm bis 300 mm hat und/oder wobei jeder Arm eine Breite in der Region der Greiffläche im Bereich von 2 mm bis 50 mm definiert.

## Revendications

1. Pincette, dans laquelle la pincette comprend une paire de bras (3, 5), les bras définissant des zones de saisie opposées respectives (11, 13) à leurs extrémités distales (9, 10), les zones de saisie (11, 13) pouvant être manipulées l'une vers l'autre lors de l'utilisation pour saisir des objets ;
dans laquelle au moins une desdites zones de saisie (11, 13) comprend :
un premier profil de saisie (39, 43), dans laquelle le premier profil de saisie (39, 43) sert à saisir des seringues et est adapté pour s'agencer de façon serrée autour du cylindre d'une seringue, dans laquelle le premier profil de saisie (39, 43) est courbe et la pincette est une pincette en plastique jetable, **caractérisée en ce que** ladite au moins une desdites zones de saisie (11, 13) comprend en outre un deuxième profil de saisie différent (41, 45) qui se présente sous la forme d'une encoche qui s'étend en travers du bras, dans laquelle le deuxième profil de saisie sert à saisir des aiguilles et est configuré de manière à s'agencer de façon serrée autour d'une aiguille, et dans laquelle le deuxième profil de saisie est situé entre le premier profil de saisie et une extrémité distale libre du bras.

2. Pincette selon la revendication 1, dans laquelle le deuxième profil de saisie (41, 45) est situé à une extrémité distale du bras.

3. Pincette selon l'une quelconque des revendications précédentes, dans laquelle le premier profil de saisie (39, 43) est situé directement vers l'intérieur du deuxième profil de saisie.

4. Pincette selon l'une quelconque des revendications précédentes, dans laquelle le premier profil de saisie (39, 43) est adapté pour s'agencer de façon serrée autour de la surface extérieure d'un objet dont la largeur ou le diamètre est compris dans la gamme de 5 mm à 20 mm, et/ou dans laquelle le deuxième profil de saisie (41, 45) est adapté pour s'agencer de façon serrée autour de la surface extérieure d'un objet dont la largeur ou le diamètre est compris dans la gamme de 0,1 mm à 1,5 mm.

5. Pincette selon l'une quelconque des revendications précédentes, dans laquelle le premier profil de saisie (39, 43) définit un canal dans la zone de saisie dont la largeur est comprise dans la gamme de 3 mm à 20 mm, et/ou dans laquelle le deuxième profil de saisie (41, 45) définit un canal dans la zone de saisie dont la largeur est comprise dans la gamme de 0,1 mm à 2 mm.

6. Pincette selon l'une quelconque des revendications précédentes, dans laquelle le premier profil de saisie (39, 43) présente une profondeur qui est comprise dans la gamme de 0,5 mm à 5 mm, et/ou dans laquelle le deuxième profil de saisie (41, 45) présente une profondeur qui est comprise dans la gamme de 0,05 mm à 2 mm.

7. Pincette selon l'une quelconque des revendications précédentes, dans laquelle les zones de saisie (11, 13) des bras comprennent des premier et deuxième profils de saisie appariés.

8. Pincette selon l'une quelconque des revendications précédentes, dans laquelle chaque bras (3, 5) comprend une région opérationnelle comprenant des moyens pour faciliter la saisie manuelle de la pincette par un utilisateur, et dans laquelle la pincette comprend en outre des moyens de garde (23, 25) qui sont situés entre la région opérationnelle et la zone de saisie, de préférence dans laquelle la pincette comprend en outre une partie de connexion distale qui s'étend entre les moyens de garde et la zone de saisie du bras.

9. Pincette selon l'une quelconque des revendications précédentes, comprenant en outre des moyens de guidage (31, 33, 35, 37) servant à guider les bras lorsque ceux-ci se déplacent l'un vers l'autre, dans laquelle les moyens de guidage comprennent des dents de guidage.

10. Pincette selon l'une quelconque des revendications précédentes, dans laquelle la pincette présente une longueur qui est comprise dans la gamme de 50 mm à 300 mm, et/ou dans laquelle chaque bras définit une largeur dans la région de la zone de saisie qui est comprise dans la gamme de 2 mm à 50 mm.
